# EUROPEAN PATENT SPECIFICATION

(11) **EP 0 770 383 B1**
(45) Date of publication and mention of the grant of the patent: **11.07.2001**
(21) Application number: 96117112.1
(22) Date of filing: 24.10.1996
(51) Int. Cl.: A61K 9/00, A61K 31/35, A61K 47/36

(54) **Ophthalmic cromolyn gel preparation**
Ophthalmische Cromolyn-Gelzubereitung
Gel ophtalmique à base de cromolyn

(30) Priority: 24.10.1995 DE 19539532
(43) Date of publication of application: 02.05.1997
(73) Proprietor: Dr. GERHARD MANN chem.-pharm. Fabrik GmbH, D-13581 Berlin (DE)
(72) Inventor: Bellmann, Günther, Dr., 13593 Berlin (DE); Claus-Herz, Gudrun, Dr., 14167 Berlin (DE)
(74) Representative: Maiwald, Walter, Dr. Dipl.-Chem.

(56) References cited:
- EP-A- 0 183 457
- EP-A- 0 447 351
- EP-A- 0 590 655
- US-A- 4 136 173
- INT. J. PHARM., vol. 53, no. 3, 1989, pages 219-225, XP000614153 V. LI ET AL.: "solution viscosity effects on the ocular disposition of cromolyn sodium in the albino rabbit"

## Description

This invention relates to ophthalmic preparations which contain, as an active agent, 1,3-bis (2-carboxycromon-5-yloxy)-2-hydroxypropane, commonly known as cromolyn or cromoglycic acid (CAS-No. 16110-51-3) and/or its salts or other derivatives, especially the (di-)sodium salt (CAS-No. 15826-37-6), also known as "DSCG".

The anti-allergenic effect of cromolyn and its salts and derivatives has been known for a long time. Thus, cromolyn also combined with other forms of pharmaceutical agents, is, inter alia, used in ophthalmology. A common application form is an aqueous solution, to be used as eyedrops, as e.g. described in EP 0 082 546 and EP 0 274 590.

A very fundamental problem in any application of eyedrop formulations is the short residence time of the preparation in the eye. By excreted tear liquid, the active agent can very quickly be washed out of the eye and removed from the eye area, so that the effective local concentration of the agent, as required for the medicinal purpose, is not reached anymore after a short time interval.

A specific problem in the use of cromolyn derivatives such as, preferably, disodiumcromoglicate or its derivatives, hereinafter referred to as "DSCG", is the eye irritation caused by the active agent, which manifests itself in a strong burning sensation in the eye. This causes increased excretion of tear liquid and increased eye rubbing by the patient. Both effects lead to increased removal of the active agent from the eye area. Thus, patients' acceptance of DSCG-containing ophthalmic preparations is low due to the eye irritation caused by the preparation.

Several different proposals have been made how to maintain the necessary effective agent concentration of DSCG in the eye for as long as possible.

Thus, RD 199 024 discloses a polymeric material insert which is to be placed between the eyelid and the eyeball, and from which DSCG diffuses continuously. This has the disadvantage that patients generally experience objects inserted like this as irritating. Also, the cost of such an insert is high.

LU 86 172 discloses a DSCG/methylcellulose composition, the viscosity of which is so high that it is not immediately washed out of the eye, and thus provides sufficient residence time of the active agent in the eye area. The gel-forming substances of this preparation must be used in relatively high concentrations (2 to 3 wt.-%) and such high concentrations are very irritating to the eye. A further disadvantage of this prior art concept is that the use of methylcellulose often leads to extremely irritating stickiness effects, especially affecting the eyelid. Cationic polymers and anionic therapeutic agent delivery systems are disclosed in EP 0 590 655.

Also, the above prior art suggestions do not solve the problem of eye irritation.

US Patent 4,136,173 discloses an aqueous ophthalmic preparation which contains, inter alia, xanthane gum and locust bean gum. The physical properties of this preparation change, reversibly, from liquid to gel-like, depending on the pH value. The liquid preparation, which has a pH value of less than 5, can be applied in drop-form into the eye, and there it is transformed into the gel-like phase as soon as the pH value has adapted to the physiological pH value in the eye. US 4,136,173 does not disclose the use of cromolyn or DSCG as the ophthalmic agent, although it provides long lists of agents that can be used together with its gel technology. This prior art is not at all concerned with the eye-irritating effects of DSCG.

German patent application DE 26 34 908 discloses the use of various gelling agents, combined with a multiplicity of active ingredients, of ophthalmic applications.

Solution viscosity effects of cromolyn sodium preparations are described in Int. J. Pharm., 1989, 53(3), 219-225.

It is an object of the instant invention to provide an application form suitable for ophthalmic preparations which contain at least one cromolyn derivative as the active agent, which does not show the above disclosed disadvantages.

It has now surprisingly been found that cromolyn derivatives, especially DSCG, do not lead to eye irritation, or only cause very little eye irritation, when they are formulated in a gel which is based on xanthane or similar natural or synthetic gums, excluding cellulose-based gels. Such gel preparations thus also avoid stickiness problems which arise when cellulose derivatives are used as thickeners.

The present invention solves the object by providing an ophthalmic gel preparation which comprises an active agent based on cromolyn or its derivatives, a synthetic or natural gum, preferably xanthane as the gel-forming agent, and, in case, further customary additives used in ophthalmic preparations. The ophthalmic gel is preferably prepared by providing a gum solution, preferably a xanthane solution, and adding to this a solution which comprises at least one cromoglicine derivative, followed by mixing. Further, active agents and other customary adjuvants used in producing such ophthalmic preparations, such as, especially, centrimid, sorbitol and EDTA can be added.

From the solution, undesirable solids may then be removed as necessary, e.g. by filtration, whereupon the solution is generally homogenised and sterilised.

The advantageous properties of the inventive preparations are especially achieved by the fact that the gel preparation comprises a synthetic or natural gum, especially xanthane.

Herein, the term "xanthane" is used for those exocellular biopolysaccharides which are formed by the microorganism Xanthomonas Campestres.

The active agent is especially preferably DSCG, the (double) sodium salt of cromolyn.

The preparation can further contain other active agents or combinations of active agents, e.g. xylomethazoline, as well as gel-forming auxiliaries, solubilisers, surface-active substances, complexing agents, disinfecting agents and/or pH adjusting agents. This list of adjuvants is only provided by way of example, and it is not final; all agents and additives which may be used in ophthalmic preparations are considered usable in the context of the invention. The selection of adjuvants is determined by the desired properties and the intended use of the preparation and this selection causes no problem to the person skilled in the art.

As the solvent, the gel preparation preferably contains water.

The production of the preparation procedes, in a preferred embodiment, according to the following description. All numerical statements herein are in grams or wt.-%:
- By dissolving of xanthane in water an approximately 1.5% gum solution is made.
- Further, an aqueous active agent salt solution is prepared to contain approximately 4.4% cromolyn approximately 7.6% sorbitol, approximately 0.02% centrimid and 0.02% EDTA.
- The ph value of the active agent solution is adjusted to approximately 6 with 0.1 N NaOH.
- The active agent salt solution is filtered to remove undesirable solids.
- Then, equal volumina of both solutions are mixed and homogenised.
- The combined solutions are autoclaved and, if necessary, evaporated solvent is replaced.

This description of the production method of the preparation is of course again only an example and not limiting. Depending on the desired properties and intended uses of the preparation, the person skilled in the art will, in the customary way, modify the composition of the preparation and/or the production method. It is e.g. possible to modify, leave out, and/or combine individual method steps, or change their sequence.

The same is true with respect to the following embodiment examples:,

### Example 1

### Composition of the ophthalmic preparation

| | |
|---|---|
| Xanthane | 13.0 g |
| Sorbitol | 38.0 g |
| Cromolyn, disodium salt | 20.0 g |
| Na-Edetate | 0.1 g |
| Centrimid | 0.1 g |
| Water | 927.13 g |
| 1N NaOH for pH adjustment | |

### Production of the ophthalmic preparation:

- Xanthane is dissolved in approximately 500 g water
- in the remaining amount of water, cromolyn centrimid, sorbitol and EDTA are dissolved one after the other
- the salt solution is filtered through a 0.2 *µ*m celluloseacetate filter (non-sterile conditions)
- then, the salt solution and the xanthane gel are combined and homogenised, the pH value is, if necessary, corrected to 6.0 with 1N NaOH
- in a glass bottle, the whole preparation is autoclaved for 20 minutes at 121°C; the amount of water which evaporates during autoclaving is thereafter replaced.

The preparation is followed by packaging the preparation in sterile 10 g polyfoil tubes.

### Example 2

### Composition of the ophthalmic preparation

| | |
|---|---|
| Xanthane | 9.0 g |
| Cromolyn, disodium salt | 20.0 g |
| Centrimid | 0.1 g |
| Sorbitol | 38.0 g |
| Na-Edetate | 0.1 g |
| 1N NaOH for pH adjustment | |
| Water | 931.13 g |

### Production of the ophthalmic preparation

- Xanthane is dissolved in approximately 500 g water
- in the remaining amount of water, cromolyn, centrimid, sorbitol and EDTA are dissolved one after the other
- the salt solution is filtered through a 0.2 *µ*m celluloseacetate filter (Sartorius, non-sterile conditions)
- then, the salt solution and the xanthane gel are combined and homogenised, the pH value is, if necessary, corrected to 6.0 with 1N NaOH
- in a glass bottle, the whole preparation is autoclaved for 20 minutes at 121°C; the amount of water which evaporates during autoclaving is thereafter replaced.

The preparation is followed by packaging the preparation in sterile 10 g polyfoil tubes.

When tested, the preparations according to Examples 1 and 2 did not produce any eye irritation worth mentioning. No stickiness effects and such were observed. Residence time and long-term concentration in the eye of the active agent conformed with the therapeutic requirements. The gel preparation was very well accepted by the test patients.

## Claims

1. Ophthalmic gel preparation comprising at least one cromolyn derivative,
**characterized in that** the gel preparation contains at least one xanthane or xanthane derivative as the gel-forming agent.

2. Preparation according to claim 1,
**characterized in that** the concentration of the gel-forming agent, especially xanthane, is between 0.1 and 5 wt.-%, preferably between 0.5 and 2.5 wt.-% and especially preferred 0.7 to 1.5 wt.-% based on the total weight of the preparation.

3. Preparation according to any preceding claim,
**characterized in that** at least one further active agent, e.g. xylomethazoline, or a combination of active agents is contained in the preparation.

4. Preparation according to any preceding claim,
**characterized in that** the preparation contains further gel-forming, stabilising, disinfecting, surface-active or complexing agents or combinations of such agents.

5. Preparation according to any preceding claim,
**characterized in that** the preparation has a pH value of from 4 to 7, preferably of approximately 6.

6. Ophthalmic gel preparation with the following composition:
| | |
|---|---|
| Xanthane gum | 0.1 - 5 wt.-%, preferably 0.7 - 1.5 wt.-% |
| Disodium cromoglicate | 0.5 - 5 wt.-%, preferably approximately 2 wt.-% |
| Sorbitol | 1 - 7 wt.-%, preferably 3 - 4.5 wt.-% |
| Centrimid | 0.001 - 0.1 wt.-%, preferably 0.008 - 0.02 wt.-% |
| Na-Edetate | 0.001 - 0.1 wt.-%, preferably 0.008 - 0.2 wt.-%. |
Sodium hydroxide solution for pH adjustment to 4 - 7, preferably to approximately 6, solvent, preferably water, up to 100%.

## Patentansprüche

1. Ophthalmisches Gelpräparat umfassend mindestens ein Cromoglycinsäurederivat, dadurch gekennzeichnet, daß das Gelpräparat mindestens ein Xanthan- oder Xanthanderivat als gelbildendes Mittel enthält.

2. Präparat gemäß Anspruch 1, dadurch gekennzeichnet, daß die Konzentration des gelbildenden Stoffes, insbesondere Xanthan, zwischen 0,5 und 5 Gew.-%, vorzugsweise zwischen 0,5 und 2,5 Gew.-% und besonders bevorzugt 0,7 bis 1,5 Gew.-%, basierend auf dem Gesamtgewicht des Präparates, beträgt.

3. Präparat nach einem der vorhergehenden Ansprüche, dadurch gekennzeichnet, daß mindestens ein weiterer Wirkstoff, zum Beispiel Xylamethazolin, oder eine Kombination von Wirkstoffen in dem Präparat enthalten ist.

4. Präparat nach einem der vorhergehenden Ansprüche, dadurch gekennzeichnet, daß das Präparat ferner gelbildende, stabilisierende, desinfizierende, oberflächenaktive oder komplexierende Stoffe oder Kombinationen derartiger Stoffe enthält.

5. Präparat nach einem der vorhergehenden Ansprüche, dadurch gekennzeichnet, daß das Präparat einen pH-Wert von 4 bis 7, vorzugsweise von ungefähr 6 aufweist.

6. Ophthalmisches Gelpräparat mit der folgenden Zusammensetzung:
| | |
|---|---|
| Xanthangummi | 0,1 bis 5 Gew.-%, vorzugsweise 0,7 bis 1,5 Gew.-% |
| Dinatriumchromoglicat | 0,5 bis 5 Gew.-%, vorzugsweise ungefähr 2 Gew.-% |
| Sorbitol | 1 bis 7 Gew.-%, vorzugsweise 3 bis 4,5 Gew.-% |
| Centrimid | 0,001 bis 0,1 Gew.-%, vorzugsweise 0,008 bis 0,02 Gew.-% |
| Natriumedetat | 0,001 bis 0,1 Gew.-%, vorzugsweise 0,008 bis 0,2 Gew.-% |
sowie Natriumhydroxidlösung zur pH-Einstellung auf 4 bis 7, vorzugsweise auf ungefähr 6, und Lösungsmittel, vorzugsweise Wasser, auf 100 %.

## Revendications

1. Gel ophtalmique comprenant au moins un dérivé de cromolyn caractérisé en ce que le gel contient au moins un xanthane ou un de ses dérivés en tant qu'agent gélifiant.

2. Gel selon la revendication 1, caractérisé en ce que la concentration de l'agent gélifiant, en particulier de xanthane, est comprise entre 0,1 et 5 % en poids de préférence compris entre 0,5 et 2,5 % en poids et préférentiellement entre 0,7 et 1,5 % en poids par rapport au poids total du gel.

3. Gel selon l'une des revendications précédentes, caractérisé en ce qu'il contient au moins un principe actif, par exemple la xylomethazoline, ou une combinaison de principe actif.

4. Gel selon l'une des revendications précédentes, caractérisé en ce qu'il contient en outre des agents gélifiant, stabilisant, désinfectant, tensio-actif ou combinaisons de ces agents.

5. Gel selon les revendications précédentes, caractérisé en ce qu'il présente un pH compris entre 4 et 7, de préférence aux environs de 6.

6. Gel ophthalmique présentant la composition suivante :
| | |
|---|---|
| Gomme de xanthane | : 0,1 à 5 %, avantageusement 0,7 à 1,5 % en poids ; |
| Cromoglicate de disodium | : 0,5 à 5 % avantageusement environ 2 % en poids ; |
| Sorbitol | : 1 à 7 %, de préférence 3 à 4,5 % en poids ; |
| Centrimid | : 0,001 à 0,1 %, de préférence 0,008 à 0,02 % en poids ; |
| Edetate de sodium | : 0,001 à 0,1 %, avantageusement 0,008 à 0,2 % en poids. |
Hydroxide de sodium pour ajuster le pH compris entre 4 et 7, de préférence environ 6, solvant, de préférence eau, jusqu'à 100 %.
